# EUROPEAN PATENT SPECIFICATION

(11) **EP 0 907 789 B1**
(45) Date of publication and mention of the grant of the patent: **09.10.2002**
(21) Application number: 97926351.4
(22) Date of filing: 03.06.1997
(51) Int. Cl.: D21F 1/00

(54) **DETERMINATION OF VOLATILE ORGANIC COMPOUNDS IN THE PRODUCTION OF PAPER OR PAPERBOARD**
ERMITTLUNG VON FLÜCHTIGEN ORGANISCHEN VERBINDUNGEN BEI DER HERSTELLUNG VON PAPIER ODER PAPPE
DETERMINATION DES COMPOSES ORGANIQUES VOLATILS LORS DE LA PRODUCTION DE PAPIER OU DE CARTON

(30) Priority: 05.06.1996 SE 9602225
(43) Date of publication of application: 14.04.1999
(73) Proprietor: Korsnäs AB (publ), 801 81 Gävle (SE)
(72) Inventor: AXRUP, Lars, S-802 69 Gävle (SE); NILSSON, Torsten, S-802 55 Gävle (SE)
(74) Representative: Onn, Thorsten
(86) International application number: SE9700966
(87) International publication number: WO97046755

(56) References cited:
- DIALOG INFORMATION SERVICES, File 240, PAPERCHEM, Dialog Accession No. 0575842, PAPERCHEM No. 65-15842, J.A.K. CRAWFORD et al., "Votatile Organic Emissions from Pulp and Paper Mill Sources; Sulfite Mills"; & NCASI, TECHNICAL BULLETIN, 682, (NEW YORK), 213 p. (November 1994).

## Description

The present invention relates to the determination of volatile organic compounds (VOC) in the production of paper or paperboard. More precisely, the invention relates to a method of predicting, during the production of pulp, paper or paperboard, the concentration of particular volatile organic components which are emitted by a finished paper or paperboard product.

### Background

Paper and paperboard products emit small amounts of volatile organic compounds which primarily derive from the wood raw material. Packages intended for food-stuff should emit as little VOC as possible, so that the food-stuff shall not be influenced by taste or odor from the package. With regard to packaging of food-stuff in so-called liquid board, a steady reduction of odor and taste influence have been achieved during the past 10-15 years.

Liquid board consists of a coated paperboard, which normally is built-up of two or more different layers. The manufacturer of the package will then laminate the liquid board with a barrier layer of e.g. polyethylene and optionally also aluminum.

VOC in the finished packaging material may migrate through the barrier layer either by the establishment of an equilibrium with regard to VOC between the paperboard and polyethylene, and between polyethylene and fluid, or through pores, so-called pinholes in the aluminum coating. In addition to those VOC which derive from the wood raw material there are also formed VOC, primarily aldehydes, during the degradation of unsaturated fatty acids in the paperboard. VOC may also be emitted by the coating layer.

Hexanal is the component of VOC that so far has shown the greatest correlation to the odor and taste influence on the liquid which is kept in the paperboard.

At present, VOC is analyzed by so-called "headspace" gas chromatography (GC) of frozen samples of paper or paperboard. The samples are not analyzed during the production process. However, this would be desirable so that the process could be controlled to minimize the amount of VOC in the finished product.

### Description of the invention

Surprisingly, it has now been found that it is possible to verify a good correlation between the concentration of several volatile organic compounds, which are significant for odor and taste influence, in the gas/vapor phase emitted by the pulp, paper or paperboard web during production, and the concentration of the same compounds emitted by the finished product.

Thus, the invention is directed to a method of predicting, during the production of pulp, including fluff pulp and fluff, paper or paperboard, the concentration of one or several particular volatile organic compounds (VOC) that are emitted by the finished pulp, paper or paperboard product, whereby the determination of the concentration of one or several particular VOC is performed in the gas/vapor phase that is emitted by the pulp, paper or paperboard web during production.

In an embodiment of the invention, the method is applied to a paper or paperboard web for the prediction of the concentration of one or several particular VOC that is (are) emitted by the finished coated and/or laminated paper or paperboard product.

The invention is not limited to any specific method of analysis, but in a preferred embodiment the determination of the concentration of one or several particular VOC is performed with the aid of infra-red spectroscopy or thermal desorption gas chromatography during the production, and the measured result obtained is compared with reference values obtained by analyses of the finished pulp, paper or paperboard product for calibration of the method.

In a particularly preferred embodiment the comparison is performed with the aid of a computer.

In an example of application, particularly in the production of liquid board, the concentration of at least one of the VOC components 1-butanol, α-pinene, carene, hexanal and nonanal is determined. Normally, the concentration of all of the mentioned components is determined. These components have been measured in concentrations of approximately 50-900 µg per kg paper or paperboard, but with the mentioned methods of analyses it is possible to analyze amounts in the order of magnitude of 1 µg per kg or even lower than that. The possibility to measure such low concentrations is of crucial importance if one desires to analyze some particular VOC which is emitted in such a low concentration from the finished product. It should be mentioned that the concentration of a group of compounds, such as aldehydes, can be determined, if desired.

In a particularly preferred embodiment of the invention the concentration of hexanal, which has proven to be a good indicator for odor and taste influence, is determined.

It should be mentioned that the determination of the concentration of VOC with the aid of thermal desorption gas chromatography can be performed in about 30 minutes, and the corresponding determination with IR-spectroscopy and computer aid can be performed practically simultaneously, in about 30 seconds.

In a further preferred embodiment of the invention the determination of the concentration is performed by using infra-red spectroscopy over a continuous spectrum from 400 cm⁻¹ to 4000 cm⁻¹, whereby data between 500 cm⁻¹ - 700 cm⁻¹, 1400 cm⁻¹ - 1950 cm⁻¹, 2100 cm⁻¹ - 2300 cm⁻¹ and 3450 cm⁻¹ - 4000 cm⁻¹, at which wavelengths water and carbon dioxide absorb heavily, are omitted.

The invention will now be disclosed in more detail with reference to specific embodiments thereof, but it should be understood that it is not limited to these embodiments.

### Programming

To facilitate the handling of results, a plurality of computer programs were written. Most of them were written in Borland™ Delfi™ under Microsoft™ Windows™ 3.11. To get the routines concerning inverting and multiplication of matrices to go faster, these were written in assembler. Other languages used to a minor extent have been Microsoft™ VisualC++™ and Microsoft™ Visual Basic™, respectively. All of the programs have been written and run on a Pentium™ 120MHz PC with 16MB RAM without any problems.

To check and develop all of the methods and algorithms, MatLab™ (The MathWorks Inc) and Mapel V release 3 (Waterloo Maple Software) were used. Simpler statistical calculations such as ANOVA have been made in Microsoft™ Excel™; the results from factor trials and response areas have been calculated in Statgrafics™ +(Statistical Grafics Corp).

### Chromatography

A system having adsorbing tube and thermal desorption-GC-FID for measurement of VOC in air was set up and optimized. To optimize the system a factor trial and a simplex method were used. The adequacy of two different adsorbents for the measurements was evaluated.

### Materials

The chromatographic system consisted of a Perkin-Elmer™ "Automatic Termal Desorption" ATD 400™, HP5790A GC and a PC with the software Baseline from Waters. The system was controlled from Baseline via a Waters™ eventbox with 8 digital outputs and 2 inputs.

### Program for the integration

A special program was developed for integration, since Baseline does not support the type of integration which was used. The integrating algorithm of the new program is based on that the integration starts and stops at a certain time, determined at the calibration, before and after the peak maximum. Considerably better reproducibility was obtained with this algorithm than with Baseline's. Baseline™ together with several other programs and integrators uses the first and second derivative of the signal for start and stoop, respectively, of the integration.

If the algorithm detects overlapping peaks, a number of gauss functions corresponding to the number of peaks are adjusted with the technique "Ordinal least squares". Other functions (peak forms) determined by calibration may also be used. This technique gives a better result when the overlapping peaks are of different size than at division by "perpendicular drop". If the peaks differ in size very much, "skimming" of the smaller peak is used.

### Filter for noise reduction

A low pass filter was inserted between the A/D-card in the computer and the electrometer of the GC to remove disturbances, above all hum, from the large number of fluorescent tubes in the lab as well as to prevent high-frequency noise from folding around the sampling-frequency. The noise suppression amounted to approximately 40dB (100 times).

### Adsorbent tube

1-50 adsorbent tubes for Perkin Elmer™ ATD400 in which VOC had been adsorbed, were placed in a carrousel. One by one the tubes are transported to a holder that connects the ends of the tube to the system. Before the desorption starts, helium is flushed through the tube in the opposite direction to the air stream during the adsorption. The tube is connected downstream a cold trap which is cooled to -30°C and is packed with a small amount of adsorbent.

After approximately 1 minute the adsorbent tube is heated to 250°C for 10 minutes, whereby VOC is eluted with the helium stream out of the tube; the tube is desorbed.

After the desorption, the adsorbent tube is disconnected from the system and the inlet to the cold trap is instead connected with the column, the helium stream being at the same time reversed and the cold trap is rapidly heated to 275°C. Thereby VOC is desorbed rapidly from the small amount of adsorbent.

In spite of the minute volume of the cold trap, the stream from this must be split since a capillary column is used. If the stream is not split, a band broadening of rapidly eluting components will undoubtedly appear, since the gas volume of the trap is not negligible compared to the flow of the column.

The ATD also offers a possibility of split prior to the cold trap. This can be utilized to prevent the trap from being saturated if a very large amount of VOC has been adsorbed in the adsorbent tube.

### Conditions of Analyses

- GC Temperature program: 60°C for 2 min. followed by 20°/min. to 220°C, 220°C for 5 min., then cooling.
- Flow of column: 4.2 ml/min.
- Flow of split: 16.8 ml/min.

### Adsorbent

An important requirement of the adsorbent is that the retention of water is low. This requirement is fulfilled by Tenax™ GR (Detlev Helmig and Lee Vierling, Anal. Chem. 1995, 67, 4380-4386).

### Packing of Adsorbent Tube

The adsorbent tubes were washed in hot water and RBS prior to the packing, rinsed in deionized water, 95% ethanol and dried in oven at 110°C. Approximately 100 mg Tenax™ TA or ~130 mg Tenax™ GR were filled into the tube when a wad of silanized glass-wool and a spring stopper had been placed in one opening of the tube. During the filling the tube was vibrated to pack the adsorbent well.

After the filling a wad of silanized glass-wool and a spring stopper were placed in the open opening. The spring stoppers came to use when the adsorbent had proven to somewhat crimp after a couple of heating and cooling cycles, which had resulted in the transportation of both glass-wool and adsorbent with the gas stream into the system of the ATD.

Three tubes were each packed with adsorbent and conditioned. When all the tubes had been cycled 25°C - 300°C for 30 min. - 25°C three times they were vibrated one more time, and one of the wads and springs was pushed into the tube due to the previously observed crimping of the adsorbent. Prior to the next experiment the adsorbent was conditioned by the tubes being cycled 25°C - 300°C for 30 min. - 25°C 10-12 time by the ADT repeating the method during 48 hours. The direction of flow which had been used at the conditioning was then used at all subsequent desorptions so that no cracks or channel should arise in the adsorbent. At adsorption "measurement" the flow is however the opposite so that VOC shall not have to be eluted through the whole of the adsorbent at desorption.

### Measurement of VOC around the Paper Making Machine and from Paper

Correlations between VOC, primarily hexanal, in the drying section of the paper making machine and VOC emitted from the finished product were investigated..

### Performance

Three measurements of VOC were performed in the later part of the drying section prior to the coating. Simultaneously with these measurements paper samples were taken out. These were freezed at >-18°C in cellophane packages for subsequent analyses. The analyzed components were 3-10 carbon atom aliphatic aldehydes, α- and β-pinene, carene, limonene and 1-butanol. To evaluate possible correlations all the concentrations were normalized in relation to their mean values. These normalized date were used for ANOVA analyses for hexanal as well as for registration of data which are shown in Tables 1 and 2.

### Calibration

Retention times for analyzed components were determined by preparing 50 µg/ml solutions of 3-10 carbon atoms aliphatic aldehydes, α- and β-pinene, carene, limonene and 1-butanol in 95% ethanol, and 5 µl of each solution and also 95% ethanol only were injected with a GC-syringe into the glass-wool at the inlet of the tube. Then 0.5 I nitrogen gas was pumped through the tube to distribute the calibration solution onto the adsorbent. The tubes were then analyzed in the system.

When the retention times had been determined, a calibration curve was created by preparation of six calibration solutions 1, 2, 5, 10, 20 and 50 µg/ml with regard to 3-10 carbon atom aliphatic aldehydes, α- and β-pinene, carene, limonene and 1-butanol in 95% ethanol. The tubes were connected with an air pump having the flow rate 50 ml/min., 5 µl of the calibration solution was injected into the glass-wool and 0.5 l nitrogen gas was pumped through.

In order to receive good reproducibility, the syringe was washed first with pure 95% ethanol, and then 2 µl 95% ethanol, 5 µl calibration solution and additionally 1 µl pure 95% ethanol were sucked into the syringe. This method gave good reproducibility as opposed to when 5 µl calibration solution was directly sucked into the syringe.

### Calibration Curve and Residual Plot were Created

To check that the sensitivity of the system had not been changed after the calibration, three adsorbent tubes were run after all the analyses with 5 µl of 5 mg/ml of solution. The reason for running these afterwards was that there should be no risk for the analytes in the calibration solution to disturb the analysis by memory effects in the cold trap. In case the sensitivity had changed significantly a corrected response factor was calculated from this run.

### Measurement of VOC in the Drying Section

The entire roll dryer is built-in in a house of aluminum so that the warm and humid air shall not come out into the rest of the building. The measurement of VOC was performed at the two last dryer rolls inside the house. The air circulation inside the house is very strong, partly because the rapidly moving web and the rotating rolls bring air with them, partly because the room is ventilated intensively in order to keep the air humidity down.

The strong air circulation implies that the measurement gives an average of VOC in the later part of the dryer. For the measurement, air is pumped through the adsorbent tubes which are packed with Tenax™ GR.

The situation of measurement is delicate on the one hand when the air temperature is high about 60°C, which results in a low retention volume especially for volatile and polar compounds, and on the other hand when the air humidity is high ≈60%, which precludes any form of cooling of the adsorbent tubes, since the water condenses on all surfaces that are colder than 45°C. The high air humidity reduces also in its self the retention volume further. To compensate for the high air humidity an air volume was selected which was half of the one which had previously been established as the maximal possible. To further assure that no break-through had occurred at some measurement two adsorbent tubes in sequence were always used.

### Performance

Every measurement was performed in triplicate, and before a tube was used it was cycled 25°C - 300°C for 30 min. - 25°C at least twice, followed by a blank run on the tube. This was done to prevent and discover possible memory effects.

Prior to the analyses began, and between every group of adsorbent tubes, a tube without adsorbent was run to verify that the cold trap and the system were clean. This was repeated also when the analyses had been performed to verify that the cold trap had been completely desorbed.

At the maximum of one hour after a sample was taken, the tubes were desorbed and analyzed according to the following scheme:
1. Empty tube
2. The three tubes at the back from the measurement of VOC in the drying section in random order.
3. Empty tube
4. The three tubes at the front with VOC from the drying section in random order.
5. Empty tube
6. Three tubes with 5 µl of 5 µg/ml of the calibration solution in random order.
7. Empty tube.
   In total 13 tubes.

### Measurement of VOC in Paper Samples

The paper must be heated in order to emit sufficient amounts of VOC in a reasonable time. The amount of VOC thus emitted is dependent on temperature and time, which results in that the desorbed amount of VOC is only a relative measure of the amount of VOC which the paper can emit. The temperature and time used in the routine analyses, and which are also used here, are 70°C for 30 minutes.

### Performance

The frozen paper samples were cut into strips ≈5 x 40 mm. Two 20 ml glass vials were filled with 5.00 g of the strips and were provided with a Teflon stopper. The vials were placed in a refrigerator for two hours so that the initial temperature would be the same at all desorptions.

The vial was taken out from the refrigerator and placed in a heating block @ 70°C for 30 min., whereupon the air was driven out through an adsorbent tube with 0.5 liters of nitrogen gas @ 100 ml/min.

Since a large amount of water is desorbed from the paper, the adsorbent tube must be heated to 60°C so that no condensation will occur.

When both of the vials had been desorbed the adsorbent tubes were analyzed as above, however with the difference that the flow to the cold trap was split 10 times and the calibration solution contained 50 µg/ml of the references.

### Results

In Table 1 and 2 the concentration of each component in relation to the average concentration of the component during the series of measurements are disclosed. The reason for this is that the absolute concentration varies considerably between the components. An ANOVA analysis was performed on the results obtained for hexanal when these had been normalized.

**Table 1**

| In the dryer measured concentration of VOC, each component being normalized against its average for the series of measurement. | | | |
|---|---|---|---|
| At the time | 26/2 8.35 | 26/2 16.15 | 27/2 16.20 |
| Hexanal | 0.72 | 0.88 | 1.40 |
| 1-Butanol | 0.79 | 1.21 | 1.00 |
| Carene | 1.31 | 1.30 | 0.39 |
| Nonanal | 0.55 | 1.34 | 1.11 |
| Decanal | 0.66 | 1.13 | 1.21 |

**Table 2**

| From the paper emitted amount of VOC, each component being normalized against its average of the series of measurement. | | | |
|---|---|---|---|
| At the time | 26/2 8.35 | 26/2 16.15 | 27/2 16.20 |
| Hexanal | 0.90 | 0.95 | 1.15 |
| 1-Butanol | 0.95 | 1.16 | 0.90 |
| Carene | 1.13 | 1.26 | 0.61 |
| Nonanal | 0.91 | 0.63 | 1.46 |
| Decanal | 0.64 | 0.92 | 1.44 |

### Conclusions

It is possible to predict the amount of VOC in the finished product from the concentration of VOC in the air in the drying section, since there is a good correlation between the concentration of hexanal in the later part of the drying section and the amount of hexanal desorbed from the paper samples. The correlation was good also for carene and 1-octanol. The rest of the analyzed components were present in too low concentrations in one or both of the samples for any conclusions to be made concerning possible correlations.

By the optimization which was performed on the system considerably better separation and sensitivity were obtained than when the parameters were chosen randomly, which was the starting point when the optimization began.

The installation of a high pass-filter in the signal path between the A/D transducer and the electrometer gave a considerable improvement of the signal-noise, partly because noise from other electrical equipment was removed and partly because high frequency noise which had previously been folded around the Nykvist frequency was removed.

The method of integration which was used with time regulated start and stop of the integration and adaptation of gauss function to the parts of the chromatographic presentations where the peaks overlap each other, gave much lower standard deviations than current methods, where the first and the second derivative are used for start and stop of the integration and "perpendicular drop" or "skimming" is used at overlapping peaks. When the peak form diverged considerably from the gauss form the adaptation could be further improved by calibration of the OLS model with reference runs.

The analyses of VOC in the drying section resulted in good accuracy and at that volume of air, 0.75 I, which was pumped through the adsorbent tubes, no problems arouse with break-through of any of the analytes.

The analyses of desorbed VOC from the paper were more difficult to perform with high accuracy, most of all because the method is not absolute but requires the paper to being desorbed during an exact period of time at an exact temperature and because the evacuation of air from the vial always is done in exactly the same way. The size and form of the paper strips and their placing in the vial may also contribute to this.

### Spectrophotometric Determination of Components in VOC

The possibility of measuring VOC directly with the help of IR spectroscopy was examined. As a pre-study a 14 cm gas cuvet was constructed for installation in the optical bench of the FT-IR equipment. In connection therewith the detection limited was determined for propanal. The FT-IR used was a FTS-60A from Biorad with appended computer system.

Spectroscopic determination of concentration is based on Lambert-Beer's law. Several methods and algorithms exist for the simultaneous determination of the concentration of several components from spectra, for example OLS "Ordinal Least Squares" and WLS "Weighted Least Squares" (Intermediate statistical methods and applications, Berenson Levine Goldstein, Prentice-Hall ISBN 0-13-470781-8), and PLS "Partial Least Squares" and PCR "Principal Component Regression" (A user's guide to principal components, J. Edward Jackson, Wiley).
The method used herein was OLS.

### Materials

- 14 cm gas cuvet modified by electric heating and with heated taps connected to the inlet and outlet.
- BioRad FTS60A FT-IR with SPC3200 (UNIX) computer.
- PC (Pentium 120) with a software of its own to perform OLS.
- For desorption and measurement of VOC over paper the above disclosed method and the above disclosed chromatographic system are used.
- Oven for heating of equipment.
- Teflon™ tubing
- 20 ml glass syringe with Teflon piston.
- Liquid paperboard for analysis.
- References of Water, Ethanol, 1-Butanol, Carene, α-pinene, β-pinene, Limonene, Hexanal and Nonanal with a purity of at least p.a.

### Performance

### Recording of reference spectra

The cuvet was connected to the optical bench of the FT-IR equipment and the instrument was mounted and adjusted as required.

A heated (80°C) and evacuated one-liter suction flask was filled with nitrogen gas, whereupon 5 µl of reference substance was injected with GC-syringe.

The cuvet was evacuated and the valve at the outlet was closed.
The inlet was connected to the suction flask via a Teflon™ tubing, whereupon the inlet valve was opened and the nitrogen gas with reference substance flowed in.

A spectrum with resolution 2 cm⁻¹ was recorded with 64 scannings of the region 400 cm⁻¹ to 4000 cm⁻¹.
As background the cuvet filled with only nitrogen gas was used. In this way spectra for Water, Ethanol, 1-Butanol, Carene, α-pinene, β-pinene, Limone, Hexanal and Nonanal were used.
The spectra were saved on a M-DOS diskette in binary format so that it could be transferred to a PC computer.

### Spectrometric Analyses of VOC from Paper Strips by OLS

The above described ATD-GC system was modified so that a Y-tube was installed before the column. The flow was then split between a deactivated silica capillary and the column instead of between the column and the split output of the ATD.

The output split of the ATD was closed and the length of the silica capillary was adjusted so that the split flow and the column flow were the same as previously. The flow out of the capillary was collected during 60 seconds directly after heating of the cold trap.

In order to collect the flow, a nitrogen gas-filled and to 80°C heated 20 ml glass syringe with teflon piston was used. The capillary was allowed to extend through a hole in the top of the oven and into the syringe to the piston. VOC and helium replace the nitrogen gas and are collected in the syringe.

To verify that all VOC was eluted out in 60 seconds, the capillary was connected to the FID and a chromatogram was registered at 60°C. In 45 seconds all the VOC had eluted out.

The paper strips were desorbed as described above and the adsorbent tube was placed in the carrousel of the ATD. The analyses was started and exactly when the heating of the cold trap began, the syringe was placed over the silica capillary for 60 seconds, and then it was taken away and plugged. The chromatographic analysis was continued while the content of the syringe was transferred to the evacuated IR cuvet and a spectrum was recorded with the resolution 2 cm⁻¹ and 256 scans.

The spectrum was then saved on a MS-DOS diskette in binary form for use in the OLS analysis. Then the result from the chromatographic analysis and from OLS were compared for five paper samples.

### OLS Analysis

OLS is based on normal equation in matrix form which approximate the concentration by minimizing the square sum of the residuals.

### 1. Modification of spectra.

To avoid possible problems with distorted base line all the spectra were corrected by being fourier-transformed to the frequency domain and the lowest frequencies were omitted. The inverse Fourier-transform was performed on the remaining components. This restores the original spectrum, but gives a straight base line and centered around zero.

All the points between 500 cm⁻¹ - 700 cm⁻¹, 1400 cm⁻¹ - 1950 cm⁻¹, 2100 cm⁻¹ - 2300 cm⁻¹ and 3450 cm⁻¹ - 4000 cm⁻¹ where water and carbon dioxide absorb heavily were omitted from the calculations.

### Results

The results from the chromatographic analysis and OLS, which are presented in Table 3, are well corresponding for 1-Butanol, Carene, α-pinene, Hexanal and Nonanal, which are present at high concentrations. However, for Ethanol, β-pinene and Limonene which are present together with some 100 other components in low concentration, the error was big.

**Table 3**

| Results from desorption of liquid paperboard, desorbed amounts measured with ATD-GC and FT-IR OLS, respectively. | | | | |
|---|---|---|---|---|
| | **ATD-GC** | | **FT-IR OLS** | |
| Component | Result µg/kg | sd | Result µg/kg | sd |
| 1-Butanol | 863 | 32 | 851 | 35 |
| α-pinene | 36 | 3.1 | 48 | 3.5 |
| Carene | 50 | 4.3 | 57 | 6.1 |
| Hexanal | 305 | 12 | 298 | 9.7 |
| Nonanal | 165 | 9.1 | 159 | 8.3 |
| β-pinene | 4.1 | 0.5 | 19 | 2.1 |
| Ethanol | 2.3 | 0.12 | 12 | 1.0 |
| Limonene | 0.9 | 0.09 | 8 | 0.6 |

### Conclusions conceming IR

The results show that it is possible to simultaneously and with good accuracy determine a number of components in VOC from paper strips with the aid of FT-IR and OLS.

Determination of components having a low concentration in the sample becomes uncertain with tendency to too high value.

In order to determine the concentration of a particular component with certainty, all the components having concentrations which are the same or higher than the component must be present in the OLS-model.

With a longer cell the ATD stage can be omitted and the VOC be measured directly without preceding concentration on adsorbent.

If two calibration spectra which are in linear relationship with each other, e.g. two spectra of the same component, are included in the model, then the OLS fails.

### Summary

1. There is a good correlation between VOC in the drying section of the papermaking machine and VOC emitted by the finished product.
2. It is possible to rapidly determine a great number of components in VOC by multivariate analyses of FT-IR spectra.
3. The multivariate method used functions for dominating components but fails for others if it is riot calibrated with care.
4. If two calibration spectra are the same the OLS fails. The method then implies inverting of a singular matrix. This can be solved by using principal component regression (PCR).

## Claims

1. Method of predicting, during the production of pulp, paper or paperboard, the concentration of one or several particular volatile organic compound(s) (VOC) which is (are) emitted by a finished pulp, paper or paperboard product,
whereby a determination of the concentration of the one or several particular VOC is performed in the gas/vapor phase that is emitted by the pulp, paper or paperboard web during production.

2. Method according to claim 1, wherein the finished paper or paperboard product is coated and/or laminated.

3. Method according to claim 1 or 2, wherein the determination is performed with the aid of infra-red spectroscopy or thermal desorption gas chromatography during the production, and the obtained result of the measurement is compared with reference values obtained by analyses of the finished pulp, paper or paperboard product for calibration of the method.

4. Method according to claim 3, wherein the comparison is performed with the aid of a computer.

5. Method according to anyone of the claims 1-4, wherein the concentration of at least one of the VOC components 1-butanol, α-pinene, carene, hexanal and nonanal is determined.

6. Method according to claim 5, wherein the concentration of hexanal is determined.

7. Method according to anyone of the claims 1-6, wherein the determination of the concentration is performed by using infra-red spectroscopy over a continuous spectrum from 400 cm⁻¹ to 4000 cm⁻¹, whereby the points between 500 cm⁻¹-700 cm⁻¹, 1400 cm⁻¹-1950 cm⁻¹, 2100 cm⁻¹-2300 cm⁻¹ and 3450 cm⁻¹-4000 cm⁻¹, at which wave lengths water and carbon dioxide absorb strongly, are omitted.

## Patentansprüche

1. Verfahren zur Vorhersage der Konzentration einer oder mehrerer bestimmter, von einem fertigen Pulpe-, Papier- oder Pappeprodukt abgegebener flüchtiger organischer Verbindungen (VOC) während der Herstellung von Pulpe, Papier oder Pappe, worin die Bestimmung der Konzentration der bestimmten flüchtigen organischen Verbindung(en) in der von der Pulpe-, Papier- oder Pappebahn während der Herstellung abgegebenen Gas-/Dampfphase erfolgt.

2. Verfahren nach Anspruch 1, worin das fertige Papieroder Pappeprodukt beschichtet und/oder laminiert ist.

3. Verfahren nach Anspruch 1 oder 2, worin die Bestimmung durch Infrarot-Spektroskopie oder Thermodesorptions-Gaschromatographie während der Herstellung erfolgt und das erhaltene Messergebnis mit Bezugswerten aus Analysen des fertigen Pulpe-, Papier- oder Pappeprodukts zwecks Kalibrierung des Verfahrens verglichen wird.

4. Verfahren nach Anspruch 3, worin der Vergleich mit Hilfe eines Rechners erfolgt.

5. Verfahren nach einem der Ansprüche 1 bis 4, worin die Konzentration mindestens einer der VOC-Komponenten 1-Butanol, α-Pinen, Caren, Hexanal und Nonanal bestimmt wird.

6. Verfahren nach Anspruch 5, worin die Hexanal-Konzentration bestimmt wird.

7. Verfahren nach einem der Ansprüche 1 bis 6, worin die Bestimmung der Konzentration mittels Infrarot-Spektroskopie über ein kontinuierliches Spektrum vom 400 cm⁻¹ bis 4000 cm⁻¹ erfolgt, wobei die Punkte zwischen 500 cm⁻¹ und 700 cm⁻¹, zwischen 1400 cm⁻¹ und 1950 cm⁻¹, zwischen 2100 cm⁻¹ und 2300 cm⁻¹ und zwischen 3450 cm⁻¹ und 4000 cm⁻¹, bei welchen Wasser und Kohlendioxid stark absorbieren, ausgelassen werden.

## Revendications

1. Procédé de prédiction, pendant la fabrication de pâte, de papier ou de carton, de la concentration d'un ou plusieurs composé(s) organique(s) volatile(s) (VOC) particulier(s) émis par un produit fini de pâte, de papier ou de carton, où la détermination de la concentration dudit ou desdits composé(s) organique(s) volatile(s) particulier(s) est effectuée dans la phase gazeuse/vapeur émise par la bande de pâte, de papier ou de carton pendant la fabrication.

2. Procédé selon la revendication 1, où le produit fini de papier ou de carton est revêtu et/ou laminé.

3. Procédé selon la revendication 1 ou 2, où la détermination est effectuée à l'aide de la spectroscopie à infrarouge ou de la chromatographie gazeuse à thermodésorption pendant la fabrication, et le résultat de mesure obtenu est comparé à des valeurs de référence obtenus par des analyses du produit de pâte, de papier ou de carton fini, aux fins de calibrage du procédé.

4. Procédé selon la revendication 3, où la comparaison est effectuée à l'aide d'un ordinateur.

5. Procédé selon l'une quelconque des revendications 1 à 4, où la concentration d'au moins un des composants de VOC 1-butanol, α-pinène, carène, hexanal et nonanal est déterminée.

6. Procédé selon la revendication 5, où la concentration de l'hexanal est déterminée.

7. Procédé selon l'une quelconque des revendications 1 à 6, où la détermination de la concentration est effectuée par la spectroscopie à l'infrarouge sur un spectre continu de 400 cm⁻¹ à 4000 cm⁻¹, les points entre 500 cm⁻¹ et 700 cm⁻¹, entre 1400 cm⁻¹ et 1950 cm⁻¹, entre 2100 cm⁻¹ et 2300 cm⁻¹ et entre 3450 cm⁻¹ et 4000 cm⁻¹, où l'eau et le dioxide de carbone absorbent fortement, étant omis.
